(19) Europäisches Patentamt
European Patent Office
Office européen des brevets

(11) Veröffentlichungsnummer: **0 142 764**
**B1**

(12) # EUROPÄISCHE PATENTSCHRIFT

(45) Veröffentlichungstag der Patentschrift: **12.08.87**

(51) Int. Cl.⁴: **C 07 C 154/00**, C 07 C 149/273, C 07 C 149/16, C 07 C 155/02

(21) Anmeldenummer: **84113273.1**

(22) Anmeldetag: **05.11.84**

(54) **Fluorierte Thiokohlensäureesterfluoride und beta-keto-trihalogenmethylthioether.**

(30) Priorität: **17.11.83 DE 3341516**

(43) Veröffentlichungstag der Anmeldung: **29.05.85 Patentblatt 85/22**

(45) Bekanntmachung des Hinweises auf die Patenterteilung: **12.08.87 Patentblatt 87/33**

(84) Benannte Vertragsstaaten: **BE CH DE FR GB IT LI NL**

(56) Entgegenhaltungen: **Keine Entgegenhaltungen**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Klauke, Erich, Dr., Eichendorffweg 8, D-5068 Odenthal (DE)**
Erfinder: **Kühle, Engelbert, Dr., von-Bodelschwingh- Strasse 42, D-5060 Bergisch Gladbach 2 (DE)**

EP 0 142 764 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue fluorierte Thiokohlensäureesterfluoride, ein neues Verfahren zur Herstellung dieser Stoffe und deren Verwendung als Zwischenprodukte zur Herstellung von Verbindungen mit herbiziden Eigenschaften.

Es sind bereits Thiokohlensäureesterfluoride der Formel

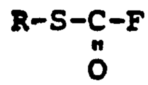

in welcher

R für Methyl, Ethyl, n-Propyl, iso-Propyl, Butyl, Phenyl, p-Chlorphenyl oder p-Fluorphenyl steht,

bekannt geworden (vgl. J. Org. Chem. 30, 1317 (1965)). Diese Verbindungen lassen sich herstellen, indem man entweder die zugrundeliegenden Mercaptane mit Fluorcarbonylchlorid umsetzt, oder indem man die entsprechenden Thiokohlensäureesterchloride mit wasserfreier Flußsäure behandelt. Nachteilig an diesen Verfahren ist, daß die jeweils benötigten Ausgangsprodukte schwierig zugänglich sind.

Es wurden jetzt neue fluorierte Thiokohlensäureesterfluoride der Formel

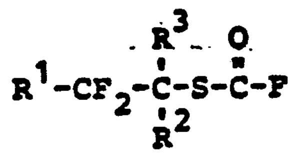

(I)

in welcher

$R^1$ für geradkettiges oder verzweigtes Alkyl mit 1 bis 12 Kohlenstoffatomen, Cycloalkyl mit 3 bis 8 Kohlenstoffatomen oder für Phenyl steht, das ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro, und

$R^2$ und $R^3$ unabhängig voneinander für Wasserstoff oder geradkettiges oder verzweigtes Alkyl mit 1 bis 4 Kohlenstoffatomen stehen, oder

$R^1$ und $R^2$ gemeinsam für eine Alkylenkette mit 3 bis 5 Kohlenstoffatomen stehen,

gefunden.

Weiterhin wurde gefunden, daß man die neuen fluorierten Thiokohlensäureesterfluoride der Formel (I) erhält, wenn man β-Ketotrihalogenmethyl-thioether der Formel

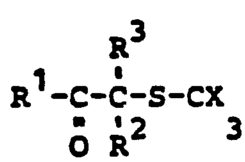

(II)

in welcher

$R^1$, $R^2$ und $R^3$ die oben angegebene Bedeutung haben und

X für Fluor oder Chlor steht, wobei mindestens ein X für Chlor steht,

mit wasserfreier Flußsäure gegebenenfalls in Gegenwart eines Lösungsmittels umsetzt.

Schließlich wurde gefunden, daß sich die neuen fluorierten Thiokohlensäureesterfluoride als Zwischenprodukte zur Herstellung von fluorierten Thiolcarbamaten, welche herbizide Eigenschaften besitzen, verwenden lassen.

Überraschenderweise zeigen die aus den erfindungsgemäßen Stoffen der Formel (I) herstellbaren fluorierten Thiolcarbamate eine bessere selektive herbizide Wirksamkeit als die konstitutionell ähnlichsten vorbekannten Verbindungen für den gleichen Verwendungszweck. Überraschend ist auch der Verlauf des erfindungsgemäßen Verfahrens. So ist zum Beispiel bekannt, daß durch Fluorierung von aliphatischen oder aromatischen Trichlormethyl-thioethern mit Fluorwasserstoff die entsprechenden Verbindungen entstehen, die in der Trichlormethyl-Gruppe partiell oder vollständig fluoriert sind (vgl. Liebigs Ann. Chem. 621, 8 (1959), FR-PS 820 796 und Chemisches Zentralblatt 1938 I, 1876). In Analogie dazu war anzunehmen, daß sich im Zuge der erfindungsgemäßen Umsetzung durch einen Austausch von Chlor gegen Fluor die entsprechenden in der-Trihalogenmethyl-Gruppe fluorierten β-Keto-trihalogenmethyl-thioether bilden würden. Im Gegensatz zu den Erwartungen entstehen jedoch die erfindungsgemäßen Stoffe der Formel (I).

Die erfindungsgemäßen fluorierten Thiokohlensäureesterfluoride sind durch die Formel (I) allgemein definiert.

Bevorzugt sind diejenigen Verbindungen der Formel (I), in denen $R^1$ für geradkettiges oder verzweigtes Alkyl mit 1 bis 8 Kohlenstoffatomen, Cycloylkyl mit 3 bis 7 Kohlenstoffatomen oder für Phenyl steht, welches ein- oder mehrfach, gleichartig oder verschieden substituiert sein kann durch Fluor, Chlor, Halogenalkyl mit 1 oder 2 Kohlenstoffatomen und 1 bis 3 Fluor- oder Chloratomen, Alkyl mit 1 bis 3 Kohlenstoffatomen, Halogenalkoxy mit 1 oder 2 Kohlenstoffatomen und 1 bis 3 Fluor- oder Chloratomen, Alkylthio mit 1 bis 3 Kohlenstoffatomen, Halogenalkylthio mit 1 oder 2 Kohlenstoffatomen und 1 bis 3 Fluor- oder Chloratomen und/oder Nitro, und die Reste $R^2$ und $R^3$ unabhängig voneinander für Wasserstoff oder geradkettiges oder verzweigtes Alkyl mit 1 bis 3 Kohlenstoffatomen stehen, oder $R^1$ und $R^2$ gemeinsam für eine Alkylenkette mit 3 oder 4 Kohlenstoffatomen stehen.

Verwendet man Acetonyl-dichlorfluormethylthioether als Ausgangsstoff und wasserfreie Flußsäure als Fluorierungsmittel, so kann der Verlauf des erfindungsgemäßen Verfahrens durch das folgende Formelschema veranschaulicht werden:

$$CH_3\text{-}CO\text{-}CH_2\text{-}S\text{-}CCl_2F \xrightarrow[-\ 2\ HCl]{+\ 2\ HF} CH_3\text{-}CF_2\text{-}CH_2\text{-}S\text{-}COF$$

Die bei dem erfindungsgemäßen Verfahren als Ausgangsstoffe benötigten β-Keto-trihalogenmethyl-thioether sind durch die Formel (II) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. X steht für Fluor oder Chlor, wobei jedoch mindestens ein X für Chlor steht.

Als Beispiele für β-Keto-trihalogenmethyl-thioether der Formel (II) seien die in den beiden folgenden Tabellen aufgeführten Stoffe erwähnt.

**Tabelle 1**

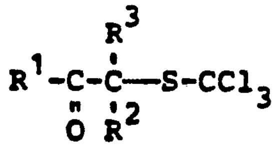

(IIa)

| $R^1$ | $R^2$ | $R^3$ |
|---|---|---|
| $CH_3$ | $CH_3$ | $CH_3$ |
| $C_2H_5$ | $CH_3$ | H |
| $C_3H_7$-n | $CH_3$ | H |
| n-$C_4H_9$- | $CH_3$ | H |
| n-$C_5H_{11}$ | H | H |
| n-$C_6H_{12}$ | H | H |
| n-$C_{12}H_{25}$ | H | H |
| 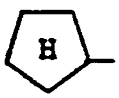 | H | H |
| 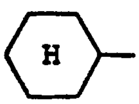 | H | H |
| 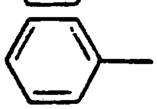 | H | H |
| 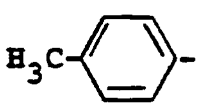 | H | H |
| 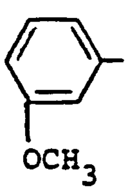 | H | H |

Tabelle 1 (Fortsetzung)

| $R^1$ | $R^2$ | $R^3$ |
|---|---|---|
| 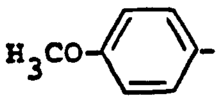 | H | H |
| 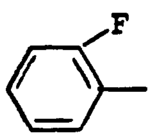 | H | H |
| 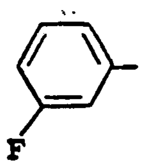 | H | H |
| 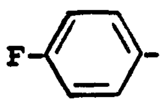 | H | H |
| 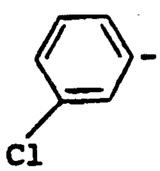 | H | H |
| 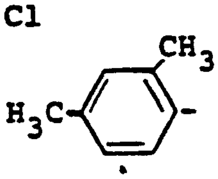 | H | H |
| 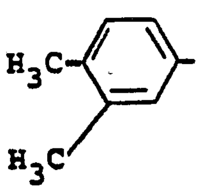 | H | H |
| 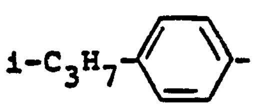 | H | H |

Tabelle 1 (Fortsetzung)

| $R^1$ | $R^2$ | $R^3$ |
|---|---|---|
| 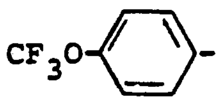 | H | H |
| 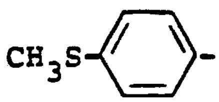 | H | H |
| 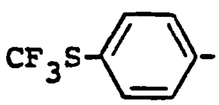 | H | H |

Tabelle 2

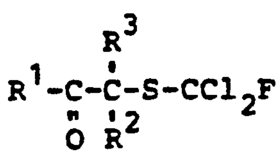

(IIb)

| $R^1$ | $R^2$ | $R^3$ |
|---|---|---|
| $CH_3$ | $CH_3$ | $CH_3$ |
| $C_2H_5$ | $CH_3$ | H |
| $C_3H_7$-n | $CH_3$ | H |
| n-$C_4H_9$- | $CH_3$ | H |
| n-$C_5H_{11}$ | H | H |
| n-$C_6H_{12}$ | H | H |
| n-$C_{12}H_{25}$ | H | H |

Tabelle 2 (Fortsetzung)

| $R^1$ | $R^2$ | $R^3$ |
|---|---|---|
| 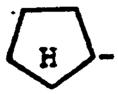 | H | H |
| 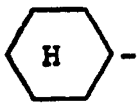 | H | H |
| 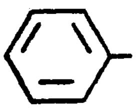 | H | H |
| 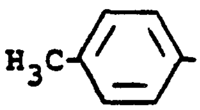 | H | H |
| 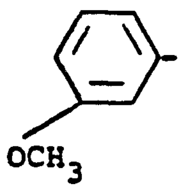 | H | H |
| 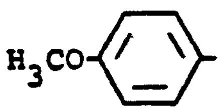 | H | H |
| 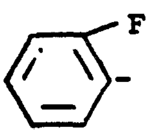 | H | H |
| 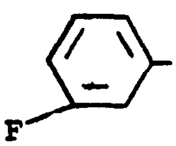 | H | H |
| 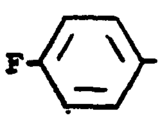 | H | H |

Tabelle 2 (Fortsetzung)

| $R^1$ | $R^2$ | $R^3$ |
|---|---|---|
| 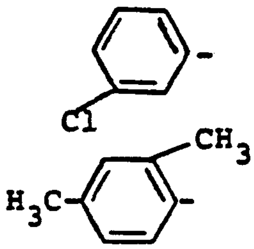 | H | H |
| | H | H |
| 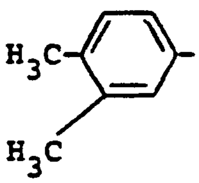 | H | H |
| 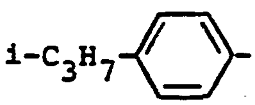 | H | H |
| 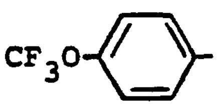 | H | H |
| 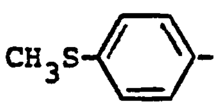 | H | H |
| 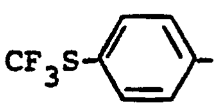 | H | H |
| 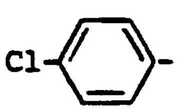 | H | H |

Tabelle 2 (Fortsetzung)

| $R^1$ | $R^2$ | $R^3$ |
|---|---|---|
| 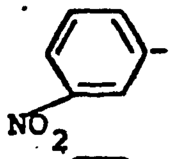 | H | H |
| 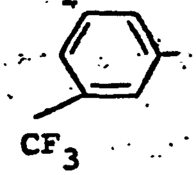 | H | H |

Die β-Keto-trihalogenmethyl-thioether der Formel (II) sind teilweise bekannt (vgl. US-PS 3 937 738 und E. Kühle "The Chemistry of the Sulfenic Acids", Georg Thieme Publ. Stuttgart, 1973, Seite 95).

Die β-Keto-trihalogenmethyl-thioether der Formel

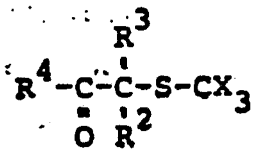

(IIc)

in welcher

$R^2$, $R^3$ und X die oben angegebene Bedeutung haben

und für Phenyl, welches ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro steht, sind bisher noch nicht beschrieben worden.

Bevorzugt sind diejenigen Verbindungen der Formel (IIc), in denen $R^4$ für Phenyl steht, welches ein- oder mehrfach, gleichartig oder verschieden substituiert sein kann durch Fluor, Chlor, Alkyl mit 1 bis 3 Kohlenstoffatomen, Halogenalkyl mit 1 oder 2 Kohlenstoffatomen und 1 bis 3 Fluor- oder Chloratomen, Alkoxy mit 1 bis 3 Kohlenstoffatomen, Halogenalkoxy mit 1 oder 2 Kohlenstoffatomen und 1 bis 3 Fluor- oder Chloratomen, Alkylthio mit 1 bis 3 Kohlenstoffatomen, Halogenalkylthio mit 1 oder 2 Kohlenstoffatomen und 1 bis 4 Fluor- oder Chloratomen und/oder Nitro. Die Reste $R^2$, $R^3$ und X haben vorzugsweise diejenigen Bedeutungen, die oben bereits als bevorzugt genannt wurden.

Die Verbindungen der Formel (IIc) lassen sich herstellen, indem man Ketone der Formel

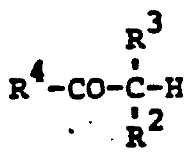

(III)

in welcher

$R^2$, $R^3$ und $R^4$ die oben angegebene Bedeutung haben,

mit Trihalogenmethyl-sulfenyl-chloriden der Formel

$Cl\text{-}S\text{-}CX_3$ (IV)

in welcher

X die oben angegebene Bedeutung hat,

gegebenenfalls in Gegenwart eines Verdünnungsmittels, wie zum Beispiel Chloroform, bei Temperaturen zwischen 0°C und 100°C umsetzt. Die Aufarbeitung erfolgt nach üblichen Methoden.

Auch die β-Keto-trihalogenmethyl-thioether der Formel

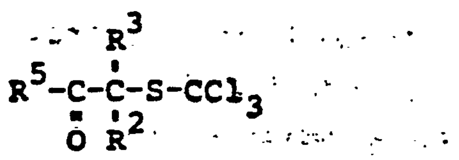

(IId)

in welcher
$R^2$ und $R^3$ die oben angegebene Bedeutung haben und
$R^5$ für geradkettiges oder verzweigtes Alkyl mit 1 bis 12 Kohlenstoffatomen oder Cycloalkyl mit 3 bis 8 Kohlenstoffatomen steht.
sind neu.

Bevorzugt sind diejenigen Verbindungen der Formel (IId), in denen $R^5$ für geradkettiges oder verzweigtes Alkyl mit 1 bis 8 Kohlenstoffatomen oder für Cycloalkyl mit 3 bis 7 Kohlenstoffatomen steht. $R^2$ und $R^3$ haben vorzugsweise diejenigen Bedeutungen, die oben bereits als bevorzugt genannt werden.

Die Verbindungen der Formel (IId) lassen sich herstellen. indem man Ketone der Formel

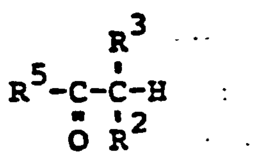

(V)

in welcher
$R^2$, $R^3$ und $R^5$ die oben angegebene Bedeutung haben,
mit Trichlormethyl-sulfenyl-chlorid der Formel
$Cl\text{-}S\text{-}CCl_3$ (IVa)
gegebenenfalls in Gegenwart eines inerten Verdünnungsmittels bei Temperaturen zwischen 0°C und 100°C umsetzt. Die Aufarbeitung erfolgt nach üblichen Methoden.

Bei dem erfindungsgemäßen Verfahren dient wasserfreie Flußsäure als Fluorierungsmittel.

Die erfindungsgemäße Umsetzung kann gegebenenfalls in Gegenwart eines Verdünnungsmittels durchgeführt werden, das unter den Reaktionsbedingungen inert ist. Vorzugsweise verwendbare Solventien sind hierbei chlorierte Kohlenwasserstoffe, wie Methylenchlorid, Chloroform und Chlorbenzol.

Die Reaktionstemperatur kann bei der Durchführung des erfindungsgemäßen Verfahrens innerhalb eines bestimmten Bereiches variiert werden. Im allgemeinen arbeitet man bei Temperaturen zwischen -20°C und +100°C, vorzugsweise zwischen -10°C und +70°C.

Die erfindungsgemäße Umsetzung kann sowohl unter Normaldruck als auch unter erhöhtem Druck vorgenommen werden. Der Überdruck kann dabei bis zu 6 bar betragen.

Die erfindungsgemäße Umsetzung wird in Apparaturen durchgeführt, die üblicherweise für Reaktionen zum Austausch von Chlor gegen Fluor gebräuchlich sind.

Bei der Durchführung des erfindungsgemäßen Verfahrens geht man im allgemeinen so vor, daß man die wasserfreie Flußsäure vorlegt und den β-Keto-trihalogenmethyl-thioether der Formel (II) bei niedriger Temperatur zugibt. Dabei wird im allgemeinen auf die Verwendung eines zusätzlichen Lösungsmittels verzichtet. Sollen jedoch kristalline Ausgangssubstanzen zugegeben werden, so geschieht dieses zweckmäßigerweise so, daß man die betreffenden Substanzen in einem unter den Reaktionsbedingungen inerten Solvens gelöst hinzugibt. Die erfindungsgemäße Umsetzung verläuft im allgemeinen bereits bei niedrigen Temperaturen recht zügig; sie ist unter drucklosen Bedingungen zumeist schon innerhalb weniger Stunden beendet. Verwendet man relativ reaktionsträge Ausgangsmaterialien, so arbeitet man zweckmäßigerweise unter erhöhtem Druck bei etwas höheren Temperaturen. Dabei ist dafür zu sorgen, daß der frei werdende Chlorwasserstoff über ein regelbares Ventil entspannt werden kann.

Die Menge der einzusetzenden Flußsäure richtet sich nach der Zahl der auszutauschenden Chloratome. Bei Verwendung von 1 Mol an β-Ketotrihalogenmethyl-thioether der Formel (II) muß pro auszutauschendem Chloratom mindestens 1 Mol an wasserfreier Flußsäure eingesetzt werden. Zweckmäßigerweise verwendet man jedoch einen Überschuß, so daß die Flußsäure gleichzeitig als Lösungsmittel fungieren kann. Im allgemeinen verwendet man beim Einsatz von 1 Mol an β-Keto-trihalogenmethylthioether der Formel (II) pro auszutauschendem Chloratom 10 bis 20 Mol Flußsäure. Ein größerer Überschuß ist jedoch nicht erschwerend für die Umsetzung.

Die Aufarbeitung des nach der Fluorierung anfallenden Reaktionsgemisches erfolgt nach üblichen Methoden. Im allgemeinen werden die Reaktionsprodukte durch Destillation isoliert. Es ist jedoch auch möglich, das Reaktionsgemisch zunächst mit einem Lösungsmittel zu extrahieren, das mit Flußsäure nur wenig mischbar ist. Derartige Lösungsmittel sind zum Beispiel Methylenchlorid, Chloroform, Tetrachlorkohlenstoff, Benzin, Benzol und Chlorbenzol.

Die nach Extraktion erhaltene organische Phase wird gegebenenfalls nach vorherigem Waschen und Trocknen eingedampft und gegebenenfalls destilliert.

Die erfindungsgemäßen fluorierten Thiokohlensäureesterfluoride können zur Herstellung von herbizid

wirksamen fluorierten Thiolcarbamaten der Formel

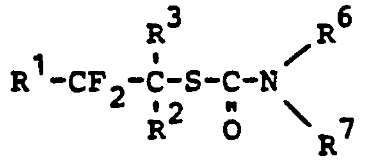

(VI)

in welcher
$R^1$, $R^2$ und $R^3$ die oben angegebene Bedeutung haben und
$R^6$ und $R^7$ unabhängig voneinander für Wasserstoff, Alkyl, Alkenyl oder Alkinyl stehen oder
$R^6$ und $R^7$ gemeinsam mit dem angrenzenden Stickstoffatom für einen gesättigten oder ungesättigten heterocyclischen Ring mit 5 bis 7 Ring-Gliedern stehen,
eingesetzt werden.

Die Herstellung der fluorierten Thiolcarbamate der Formel (VI) erfolgt dabei in der Weise, daß man fluorierte Thiokohlensäureesterfluoride der Formel

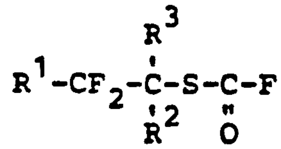

(I)

in welcher
$R^1$, $R^2$ und $R^3$ die oben angegebene Bedeutung haben, mit Verbindungen der Formel

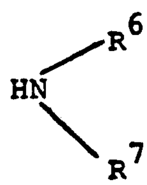

(VII)

in welcher
$R^6$ und $R^7$ die oben angegebene Bedeutung haben,
gegebenenfalls in Gegenwart eines Verdünnungsmittels sowie gegebenenfalls in Gegenwart eines Säurebindemittels bei Temperaturen zwischen 0°C und 100°C umsetzt.

Die fluorierten Thiolcarbamate der Formel (VI) besitzen sehr gute herbizide, insbesondere selektiv-herbizide Eigenschaften. Sie lassen sich besonders gut zur selektiven Bekämpfung von Cyperaceen einsetzen.

Die Herstellung der erfindungsgemäßen fluorierten Thiokohlensäureesterfluoride der Formel (I) und deren Verwendung als Zwischenprodukte zur Synthese von fluorierten Thiolcarbamaten der Formel (VI) geht aus der folgenden Beispielen hervor.

**Beispiel 1**

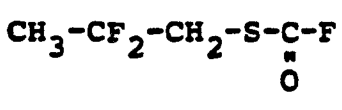

(I-1)

In einem Reaktionsgefäß aus rostfreiem Stahl wurden 200 ml wasserfreie Flußsäure Vorgelegt. Bei einer Temperatur von -10°C ließ man innnerhalb von 30 Minuten 200 g (1,05 Mol) Acetonyl-dichlorfluormethylthioether unter Rühren zutropfen, wobei sofort eine Chlorwasserstoffentwicklung einsetzte. Man erwärmte das Reaktionsgemisch bis auf 0°C und rührte bei dieser Temperatur noch so lange nach, bis praktisch keine Gasentwicklung mehr beobachtet wurde. Zur anschließenden Aufarbeitung wurde das Reaktionsgemisch fraktioniert destilliert. Man erhielt auf diese Weise 109 g (66 % der Theorie) an 2,2-Difluorpropanthiolkohlensäureesterfluorid.

Kp. 53°C/100 mbar

$n_D^{20}$ 1, 3907

Aus dem Nachlauf konnte durch Feindestillation das 2-Fluor-2-chlor-propanthiolkohlensäureesterfluorid der Formel

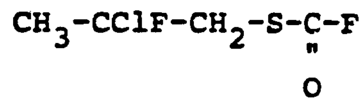

isoliert werden.

Kp. 74-75°C / 100 mbar

$n_D^{20}$ 1 4320

Nach der im Beispiel 1 angegebenen Methode wurden auch die in der folgenden Tabelle 3 formelmäßig aufgeführten Stoffe hergestellt.

**Tabelle 3**

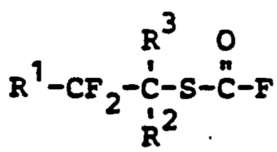

(I)

| Beispiel-Nr. | $R^1$ | $R^2$ | $R^3$ | Siedepunkt / Brechungsindex |
|---|---|---|---|---|
| 2 | $CH_3$- | $CH_3$- | H | 59°C / 100 mbar<br>$n_D^{20}$ = 1,3950 |
| 3 | $CH_3$- | $C_2H_5$- | H | 76°C / 100 mbar<br>$n_D^{20}$ = 1,4042 |
| 4 | 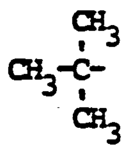 | H | H | 66°C / 20 mbar<br>$n_D^{20}$ = 1,4160 |
| 5 | n-$C_3H_7$-n | $C_2H_5$- | H | 75°C / 19 mbar<br>$n_D^{20}$ = 1,4148 |
| 6 | $CH_3$- | $CH_3$- | $CH_3$- | 72°C / 50 mbar<br>$n_D^{20}$ = 1,4128 |
| 7 | -$(CH_2)_3$ - | | H | 55 - 57 °C/16 mbar |

**Beispiel 8**

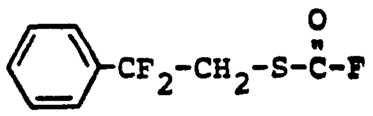

(I-8)

In einem Reaktionsgefäß aus rostfreiem Stahl wurden 300 ml wasserfreie Flußsäure vorgeregt. Bei einer Temperatur von -8°C ließ man innerhalb von einer Stunde 530 g (2,09 Mol) (Fluordichlormethyl-mercaptomethyl)-phenylketon unter Rühren zutropfen. Die dabei einsetzende Chlorwasserstoff-Entwicklung klang nach etwa einer Stunde ab und wurde durch Vierstündiges Nachrühren bei Raumtemperatur ganz beendet. Zur anschließenden Aufarbeitung wurde das Reaktionsgemisch einer fraktionierten Destillation unter vermindertem Druck unterworfen. Man erhielt auf diese Weise 342 g (74 % der Theorie) an β-Phenyl-β,β-difluorethanthiol-kohlensäureesterfluorid.

Kp = 106°C / 17 mbar
$n_D^{20}$ = 1,4891

Herstellung des Ausgangsproduktes der Formel

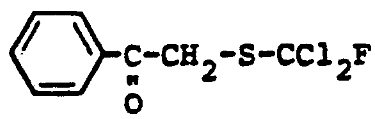

(II-1)

240 g (2 Mol) Acetophenon wurden in 300 ml Chloroform gelöst und nach Zugabe von 3 ml Ethanol und 109 g (0,64 Mol) Fluordichlormethan-sulfenchlorid bis zur Rückflußtemperatur aufgeheizt. Innerhalb von 30 Minuten ließ man noch 400 g (2,36 Mol) an Fluordichlormethan-sulfenchlorid zutropfen und erhitzte dann 5 Stunden unter Rückfluß. Zur anschließenden Aufarbeitung wurde das Reaktionsgemisch einer fraktionierten Destillation unter Vermindertem Druck unterworfen. Nach Vorläufen von unverändertem Ausgangsmaterial erhielt man 126 g an (Fluordichlormethyl-mercaptomethyl)-phenyl-keton in Form einer Flüssigkeit vom Siedepunkt 128°C/0,4 mbar. Brechungsindex: $n_D^{20}$ = 1,5653

Die Ausbeute errechnet sich zu 87 %, bezogen auf umgesetztes Acetophenon.

Das Reaktionsprodukt kann auch ohne zusätzliche Reinigung nach Abtrennung des unveränderten Acetophenons für die Fluorierung mit Flußsäure eingesetzt werden.

**Beispiel 9**

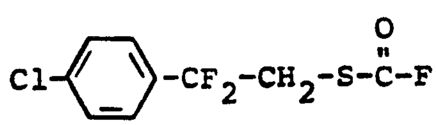

(I-9)

In einem Reaktionsgefäß aus rostfreiem Stahl wurden 300 ml wasserfreie Flußsäure vorgelegt. Bei einer Temperatur von -8°C ließ man innerhalb von einer Stunde 370 g (1,29 Mol) (4-Chlorphenyl)-(fluordichlormethyl-mercaptomethyl)-keton unter Rühren zutropfen. Nach dem Abklingen der zunächst sehr zügig einsetzenden Chlorwasserstoff-Entwicklung ließ man die Temperatur der Reaktionsmischung langsam auf Raumtemperatur ansteigen und rührte bis zur Beendigung der Chlorwasserstoff-Entwicklung. Zur anschließenden Aufarbeitung wurde das Reaktionsgemisch einer fraktionierten Destillation unter vermindertem Druck unterworfen. Man erhielt auf diese Weise 211 g (64,1 % der Theorie) an β-(4-Chlor-phenyl)-β,β-difluormethanthiol-kohlensäureester-fluorid in Form einer Flüssigkeit.

Kp = 126°C/16 mbar
$n_D^{20}$ = 1,5083

Aus dem Nachlauf wurden noch 32 g an β-(4-Chlor-phenyl)-β,β-difluorethanthiol-kohlensäuresster-chlorid der Formel

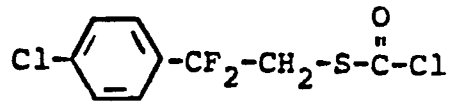

isoliert.
Kp = 145 - 146° C/12 mbar
Fp = 45° C.

**Beispiel 10**

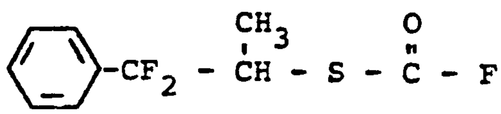
(I- 10)

Nach der in den Beispielen 8 und 9 angegebenen Methode wurde auch das (2-Phenyl-2,2-difluor-1-methyl)-ethanthiol-kohlensäureester-fluorid hergestellt.
Kp = 107° C/14 mbar
$n_D^{20}$ = 1,4839

**Beispiel 11**

Synthese des Ausgangsproduktes der Formel

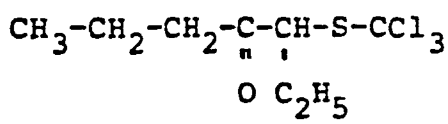
(II-2)

Ein Gemisch aus 171 g (1,5 Mol) Di-n-propylketon und 186 g (1 Mol) Perchlormethylmercaptan wurde 14 Stunden auf 100° C erhitzt. Dabei beobachtete man eine schwache, aber regelmäßige Chlorwasserstoff-Entwicklung: Zur anschließenden Aufarbeitung wurde das Reaktionsgemisch einer fraktionierten Destillation unter vermindertem Druck unterworfen. Man erhielt auf diese Weise 80 g eines Produktes, das im wesentlichem aus dem β-Keto-trichlormethylthioether der Formel (II-2) bestand, jedoch noch durch die Substanz der Formel

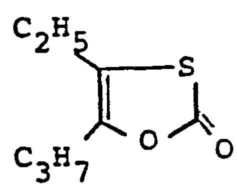

verunreinigt war.
Kp = 109° C/ 0,1 mbar
$n_D^{20}$ = 1,5156
Bei der Umsetzung mit Flußsäure bildet sich aus dem oben angegebenen Substanzgemisch die im Beispiel 5 beschriebene Verbindung.

**Beispiel 12**

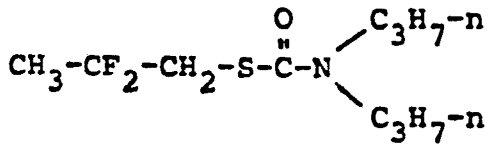

(VI-1)

Zu einer Lösung von 15,8 g (0,1 Mol) 2,2-Difluorpropan-thiol-kohlensäureesterfluorid in 100 ml Toluol wurden unter Rühren und Kühlung bei 20-28°C insgesamt 20,2 g (0,2 Mol) Di-n-propylamin zugetropft. Das Reaktionsgemisch wurde weitere 30 Minuten bei Raumtemperatur gerührt und dann mit Wasser gewaschen. Nach dem Trocknen der organischen Phase über Natriumsulfat wurde das Lösungsmittel unter vermindertem Druck abgezogen und der verbleibende Rückstand einer fraktionierten Vakuumdestillation unterworfen. Man erhielt auf diese Weise 21 g (83 % der Theorie) an N,N-Di-n-propyl-(2,2-difluorpropyl)-thiolcarbamidsäureester in Form einer Flüssigkeit vom Siedepunkt 125°C / 12 mbar.

Nach der im Beispiel 12 angegebenen Methode wurden auch die in der folgenden Tabelle 4 formelmäßig aufgeführten Stoffe hergestellt.

Tabelle 4

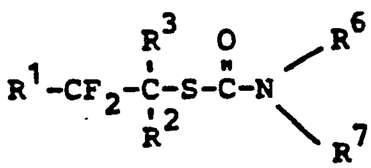

(VI)

| Bsp.-Nr. | $R^1$ | $R^2$ | $R^3$ | $R^6$ | $R^7$ | Physik. Konstante |
|---|---|---|---|---|---|---|
| 13 | $CH_3$ | H | H | $CH_3$ | H | Kp. 113°C/12 mbar |
| 14 | $CH_3$ | $CH_3$ | H | $C_2H_5$ | $C_2H_5$ | Kp. 113-115°C/ 18 mbar |
| 15 | $n\text{-}C_3H_7$ | $C_2H_5$ | H | $-(CH_2)_4-$ | | $n_D^{20} = 1,4808$ |
| 16 | 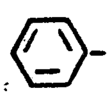 | $C_2H_5$ | H | $-(CH_2)_4-$ | | $n_D^{20} = 1,5414$ |
| 17 | $CH_3-$ | $CH_3$ | H | $-(CH_2)_4-$ | | Kp. 142 - 144°C/ 18 mbar |

Die sehr gute herbizide Wirksamkeit der fluorierten Thiolcarbamate der Formel (VI) wird durch das folgende Verwendungsbeispiel veranschaulicht.

In diesem Verwendungsbeispiel wurde die nachstehend angegebene Verbindung als Vergleichssubstanz eingesetzt:

(A) = 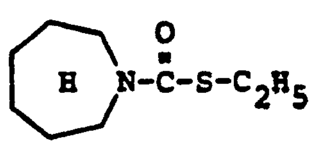

Hexahydro-1-H-azepin-1-carbamidsäure-thiolethylester (bekannt aus US-PS 3 198 786).

**Beispiel A**

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigte die Verbindung gemäß Beispiel 17 bei der Bekämpfung von Cyperus und Setaria in Mais und Baumwolle eine deutlich bessere selektive herbizide Wirksamkeit als die Vergleichssubstanz (A).

**Patentansprüche**

1. Fluorierte Thiokohlensäureesterfluoride der Formel

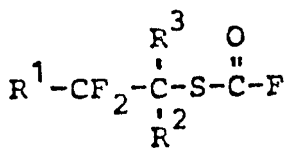

(I)

in welcher

$R^1$ für geradkettiges oder verzweigtes Alkyl mit 1 bis 12 Kohlenstoffatomen, Cycloalkyl mit 3 bis 8 Kohlenstoffatomen oder für Phenyl steht, das ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro, und

$R^2$ und $R^3$ unabhängig voneinander für Wasserstoff oder geradkettiges oder verzweigtes Alkyl mit 1 bis 4 Kohlenstoffatomen stehen, oder

$R^1$ und $R^2$ gemeinsam für eine Alkylenkette mit 3 bis 5 Kohlenstoffatomen stehen.

2. Verfahren zur Herstelluns von fluorierten Thiokohlensäureesterfluoriden der Formel

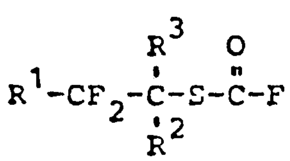

(I)

in welcher

$R^1$ für geradkettiges oder verzweigtes Alkyl mit 1 bis 12 Kohlenstoffatomen, Cycloalkyl mit 3 bis 8 Kohlenstoffatomen oder für Phenyl steht, das ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro, und

$R^2$ und $R^3$ unabhängig voneinander für Wasserstoff oder geradkettiges oder verzweigtes Alkyl mit 1 bis 4 Kohlenstoffatomen stehen, oder

$R^1$ und $R^2$ gemeinsam für eine Alkylenkette mit 3 bis 5 Kohlenstoffatomen stehen,

dadurch gekennzeichnet, daß man β-Keto-trihalogenmethyl-thioether der Formel

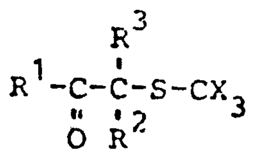

(II)

in welcher
$R^1$, $R^2$ und $R^3$ die oben angegebene Bedeutung haben und
X für Fluor oder Chlor steht, wobei mindestens ein X für Chlor steht,
mit wasserfreier Flußsäure, gegebenenfalls in Gegenwart eines Verdünnungsmittels, umsetzt.

3. Verwendung von fluorierten Thiokohlensäureesterfluoride der Formel (I) als Zwischenprodukte zur Herstellung von fluorierten Thiolcarbamaten mit herbiziden Eigenschaften.

4. β-Keto-trihalogenmethyl-thioether der Formel

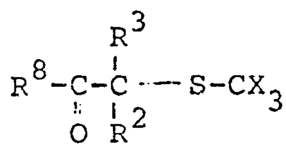

in welcher
X für Fluor oder Chlor steht, wobei mindestens ein X für Chlor steht,
$R^2$ und $R^3$ unabhängig voneinander für Wasserstoff oder geradkettiges oder verzweigtes Alkyl mit 1 bis 4 Kohlenstoffatomen stehen,
$R^8$ für Phenyl, welches ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro, und
$R^8$ für geradkettiges oder verzweigtes Alkyl mit 1 bis 12 Kohlenstoffatomen oder für Cycloalkyl mit 3 bis 8 Kohlenstoffatomen steht, wenn alle X für Chlor stehen.

5. ß-Keto-trihalogenmethyl-thioether der Formel

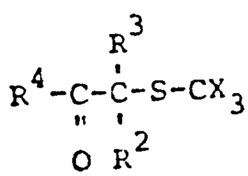

(IIc)

in welcher
$R^2$ und $R^3$ unabhängig voneinander für Wasserstoff oder geradkettiges oder verzweigtes Alkyl mit 1 bis 4 Kohlenstoffatomen stehen,
$R^4$ für Phenyl, welches ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatomen und/oder Nitro, und
X für Fluor oder Chlor steht, wobei mindestens ein X für Chlor steht.

6. Verfahren zur Herstellung von β-Keto-trihalogenmethylthioethern der Formel

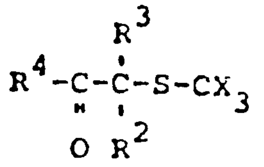

(II c)

in welcher

$R^2$ und $R^3$ unabhängig voneinander für Wasserstoff oder geradkettiges oder verzweigtes Alkyl mit 1 bis 4 Kohlenstoffatomen stehen,

$R^4$ für Phenyl, welches ein- oder mehrfach, gleichartig oder verschieden substituiert sein kann durch Halogen, Alkyl mit 1 bis 4 Kohlenstoffatomen, Halogenalkyl mit 1 bis 4 Kohlenstoffatomen und 1 bis 5 Halogenatomen, Alkoxy mit 1 bis 4 Kohlenstoffatomen, Halogenalkoxy mit 1 bis 4 Kohlenstoffatomen und 1 bis 5 Halogenatomen, Alkylthio mit 1 bis 4 Kohlenstoffatomen, Halogenalkylthio mit 1 bis 4 Kohlenstoffatomen und 1 bis 5 Halogenatome und/oder Nitro steht,

X für Fluor oder Chlor steht, wobei mindestens Ein X für Fluor steht,

dadurch gekennzeichnet, daß man Ketone der Formel

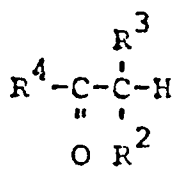

(III)

in welcher

$R^2$, $R^3$ und $R^4$ die oben angegebene Bedeutung haben, mit Trihalogenmethyl-sulfenyl-chloriden der Formel

$Cl\text{-}SCX_3$ (IV)

in welcher

X die oben angegebene Bedeutung hat,

gegebenenfalls in Gegenwart eines Verdünnungsmittels umsetzt.

7. β-Keto-trihalogenmethyl-thioether der Formel

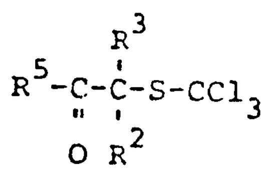

(II d)

in welcher

$R^2$ und $R^3$ unabhängig voneinander für Wasserstoff oder geradkettiges oder verzweigtes Alkyl mit 1 bis 4 Kohlenstoffatomen stehen und

$R^5$ für geradkettiges oder verzweigtes Alkyl mit 1 bis 12 Kohlenstoffatomen oder Cycloalkyl mit 3 bis 8 Kohlenstoffatomen steht.

8. Verfahren zur Herstellung von β-Keto-trihalogenmethyl-thioethern der Formel

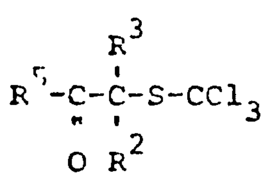

(II d)

in welcher

$R^2$ und $R^3$ unabhängig voneinander für Wasserstoff oder geradkettiges oder verzweigtes Alkyl mit 1 bis 4

Kohlenstoffatomen stehen und

$R^5$ für geradkettiges oder verzweigtes Alkyl mit 1 bis 12 Kohlenstoffatomen oder Cycloalkyl mit 3 bis 8 Kohlenstoffatomen steht,

dadurch gekennzeichnet, daß man Ketone der Formel

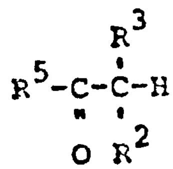

(V)

in welcher

$R^2$, $R^3$ und $R^5$ die oben angegebene Bedeutung haben,

mit Trichlormethyl-sulfenyl-chlorid der Formel

$Cl-S-CCl_3$ (IVa)

gegebenenfalls in Gegenwart eines inerten Verdünnungsmittels umsetzt.

## Claims

1. Fluorinated thiocarbonic acid ester-fluorides of the formula

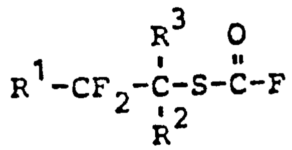

(I)

in which

$R^1$ represents straight-chain or branched alkyl with 1 to 12 carbon atoms, cycloalkyl with 3 to 8 carbon atoms or phenyl, which can be monosubstituted or polysubstituted by identical or different substituents from the group comprising halogen, alkyl with 1 to 4 carbon atoms, halogenoalkyl with 1 to 4 carbon atoms and 1 to 5 halogen atoms, alkoxy with 1 to 4 carbon atoms, halogenoalkoxy with 1 to 4 carbon atoms and 1 to 5 halogen atoms, alkylthio with 1 to 4 carbon atoms, halogenoalkylthio with 1 to 4 carbon atoms and 1 to 5 halogen atoms and/or nitro, and

$R^2$ and $R^3$ independently of one another represent hydrogen or straight-chain or branched alkyl with 1 to 4 carbon atoms or

$R^1$ an $R^2$ together represent an alkylene chain with 3 to 5 carbon atoms.

2. Process for the preparation of fluorinated thiocarbonic acid ester-fluorides of the formula

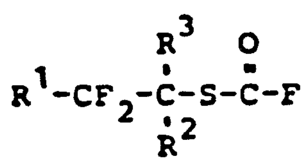

(I)

in which

$R^1$ represents straight-chain or branched alkyl with 1 to 12 carbon atoms, cycloalkyl with 3 to 8 carbon atoms or phenyl, which can be monosubstituted or polysubstituted by identical or different substituents from the group comprising halogen, alkyl with 1 to 4 carbon atoms, halogenoalkyl with 1 to 4 carbon atoms and 1 to 5 halogen atoms, alkoxy with 1 to 4 carbon atoms, halogenoalkoxy with 1 to 4 carbon atoms and 1 to 5 halogen atoms, alkylthio with 1 to 4 carbon atoms, halogenoalkylthio with 1 to 4 carbon atoms and 1 to 5 halogen atoms and/or nitro, and

$R^2$ and $R^3$ independently of one another represent hydrogen or straight-chain or branched alkyl with 1 to 4 carbon atoms, or

$R^1$ and $R^2$ together represent an alkylene chain with 3 to 5 carbon atoms,

characterised in that β-keto-trihalogenomethyl-thioethers of the formula

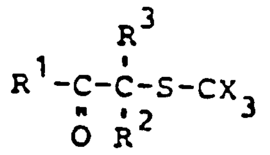 (II)

in which
$R^1$, $R^2$ and $R^3$ have the abovementioned meaning and
X represents fluorine or chlorine, at least one X representing chlorine,
are reacted with anhydrous hydrofluoric acid, if appropriate in the presence of a diluent.

3. Use of fluorinated thiocarbonic acid esterfluorides of the formula (I) as intermediates for the preparation of fluorinated thiolcarbamates having herbicidal properties.

4. β-Keto-trihalogenomethyl-thioethers of the formula

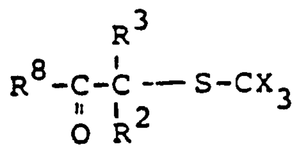

in which
X represents fluorine or chlorine, at least one X representing chlorine,
$R^2$ and $R^3$ independently of one another represent hydrogen or straight-chain or branched alkyl with 1 to 4 carbon atoms,
$R^8$ represents phenyl which can be monosubstituted or polysubstituted by identical or different substituents from the group comprising halogen, alkyl with 1 to 4 carbon atoms, halogenoalkyl with 1 to 4 carbon atoms and 1 to 5 halogen atoms, alkoxy with 1 to 4 carbon atoms, halogenalkoxy with 1 to 4 carbon atoms and 1 to 5 halogen atoms, alkylthio with 1 to 4 carbon atoms, halogenoalkylthio with 1 to 4 carbon atoms and 1 to 5 halogen atoms and/or nitro, and
$R^8$ represents straight-chain or branched alkyl with 1 to 12 carbon atoms or cycloalkyl with 3 to 8 carbon atoms when all the X's represent chlorine.

5. β-Keto-trihalogenomethyl-thioethers of the formula

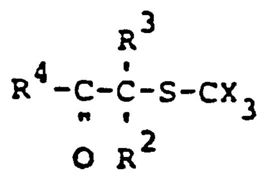 (IIc)

in which
$R^2$ and $R^3$ independently of one another represent hydrogen or straight-chain or branched alkyl with 1 to 4 carbon atoms,
$R^4$ represents phenyl which can be monosubstituted or polysubstituted by identical or different substituents from the group comprising halogen, alkyl with 1 to 4 carbon atoms, halogenoalkyl with 1 to 4 carbon atoms and 1 to 5 halogen atoms, alkoxy with 1 to 4 carbon atoms, halogenoalkoxy with 1 to 4 carbon atoms and 1 to 5 halogen atoms, alkylthio with 1 to 4 carbon atoms, halogenoalkylthio with 1 to 4 carbon atoms and 1 to 5 halogen atoms and/or nitro, and
X represents fluorine or chlorine, at least one X representing chlorine.

6. Process for the preparation of β-keto-trihalogenomethyl-thioethers of the formula

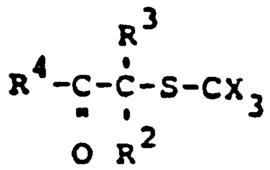

(II c)

in which

$R^2$ and $R^3$ independently of one another represent hydrogen or straight-chain or branched alkyl with 1 to 4 carbon atoms,

$R^4$ represents phenyl which can be monosubstituted or polysubstituted by identical or different substituents from the group comprising halogen, alkyl with 1 to 4 carbon atoms, halogenoalkyl with 1 to 4 carbon atoms and 1 to 5 halogen atoms, alkoxy with 1 to 4 carbon atoms, halogenoalkoxy with 1 to 4 carbon atoms and 1 to 5 halogen atoms, alkylthio with 1 to 4 carbon atoms, halogenoalkylthio with 1 to 4 carbon atoms and 1 to 5 halogen atoms and/or nitro,

X represents fluorine or chlorine, at least one X representing fluorine,.

characterised in that ketones of the formula

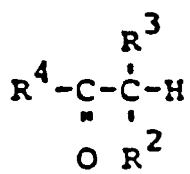

(III)

in which

$R^2$, $R^3$ and $R^4$ have the abovementioned meaning, are reacted with trihalogenomethyl-sulphenyl chlorides of the formula

$Cl\text{-}SCX_3$ (IV)

in which

X has the abovementioned meaning, if appropriate in the presence of a diluent.

7. β-Keto-trihalogenomethyl-thioethers of the formula

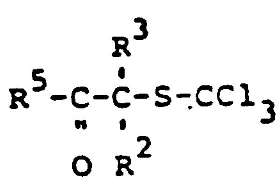

(II d)

in which

$R^2$ and $R^3$ independently of one another represent hydrogen or straight-chain or branched alkyl with 1 to 4 carbon atoms and

$R^5$ represents straight-chain or branched alkyl with 1 to 12 carbon atoms or cycloalkyl with 3 to 8 carbon atoms.

8. Process for the preparation of β-keto-trihalogenomethyl-thioethers of the formula

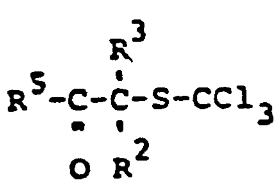

(II d)

in which

$R^2$ and $R^3$ independently of one another represent hydrogen or straight-chain or branched alkyl with 1 to 4

carbon atoms and

$R^5$ represents straight-chain or branched alkyl with 1 to 12 carbon atoms or cycloalkyl with 3 to 8 carbon atoms,

characterised in that ketones of the formula

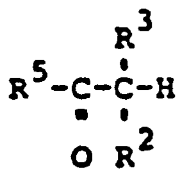

(V)

in which

$R^2$, $R^3$ and $R^5$ have the abovementioned meaning, are reacted with trichloromethyl-sulphenyl chloride of the formula

$Cl\text{-}S\text{-}CCl_3$ (IVa)

if appropriate in the presence of an inert diluent.

**Revendications**

1. Fluorures-esters thiocarboniques fluorés de formule

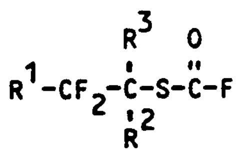

(I)

dans laquelle

$R^1$ représente un groupe alkyle à chaîne droite ou ramifiée en $C_1$-$C_{12}$, un groupe cycloalkyle en $C_3$-$C_8$ ou un groupe phényle, qui peut porter un ou plusieurs substituants indentiques ou différents choisis parmi les halogènes, les groupes alkyle en $C_1$-$C_4$, halogénoalkyle contenant 1 à 4 atomes de carbone et 1 à 5 atomes d'halogènes, alcoxy en $C_1$-$C_4$, halogénoalcoxy contenant 1 à 4 atomes de carbone et 1 à 5 atomes d'halogènes, alkylthio en $C_1$-$C_4$, halogénoalkylthio contenant 1 à 4 atomes de carbone et 1 à 5 atomes d'halogènes, et/ou nitro, et

$R^2$ et $R^3$ représentent chacun, indépendamment l'un de l'autre, l'hydrogène ou un groupe alkyle à chaîne droite ou ramifiée en $C_1$-$C_4$, ou bien $R^1$ et $R^2$ forment ensemble une chaîne alkylène en $C_3$-$C_5$.

2. Procédé de préparation des fluorures-esters thiocarboniques fluorés de formule

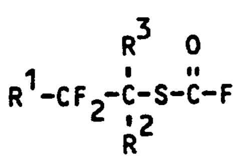

(I)

dans laquelle

$R^1$ représente un groupe alkyle à chaîne droite ou ramifiée en $C_1$-$C_{12}$, un groupe cycloalkyle en $C_3$-$C_8$ ou un groupe phényle, qui peut porter un ou plusieurs substituants indentiques ou différents choisis parmi les halogènes, les groupes alkyle en $C_1$-$C_4$, halogénoalkyle contenant 1 à 4 atomes de carbone et 1 à 5 atomes d'halogènes, alcoxy en $C_1$-$C_4$, halogénoalcoxy contenant 1 à 4 atomes de carbone et 1 à 5 atomes d'halogènes, alkylthio en $C_1$-$C_4$, halogénoalkylthio contenant 1 à 4 atomes de carbone et 1 à 5 atomes d'halogènes, et/ou nitro, et

$R^2$ et $R^3$ représentent chacun, indépendamment l'un de l'autre, l'hydrogène ou un groupe alkyle à chaîne droite ou ramifiée en $C_1$-$C_4$, ou bien $R^1$ et $R^2$ représentent ensemble une chaîne alkylène en $C_3$-$C_5$,

caractérisé en ce que l'on fait réagir des thioéthers bêta-céto-trihalogénométhyliques de formule

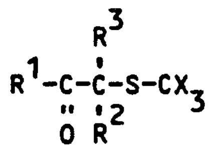

(II)

dans laquelle

$R^1$, $R^2$ et $R^3$ ont les significations indiquées ci-dessus et X représente le fluor ou le chlore, l'un au moins des symboles X représentant le chlore,

avec le fluorure d'hydrogène anhydre, éventuellement en présence d'un diluant.

3. Utilisation des fluorures-esters thiocarboniques fluorés de formule I en tant que produits intermédiaires de la préparation de thiolcarbamates fluorés possédant des propriétés herbicides.

4. Thioéthers bêta-céto-trihalogénométhyliques de formule

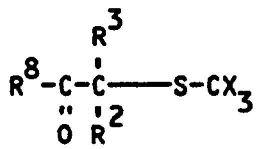

dans laquelle

X représente le fluor ou le chlore, l'un au moins des symboles X représentant le chlore,

$R^2$ et $R^3$ représentent chacun, indépendamment l'un de l'autre, l'hydrogène ou un groupe alkyle à chaîne droite ou ramifiée en $C_1$-$C_4$,

$R^8$ représente un groupe phenyle portant éventuellement un ou plusieurs substituants identiques ou différents choisis parmi les halogènes, les groupes alkyle en $C_1$-$C_4$, halogénoalkyle contenant 1 à 4 atomes de carbone et 1 à 5 atomes d'halogènes, alcoxy en $C_1$-$C_4$, halogènoalcoxy contenant 1 à 4 atomes de carbone et 1 à 5 atomes d'halogènes, alkylthio en $C_1$-$C_4$, halogénoalkylthio contenant 1 à 4 atomes de carbone et 1 à 5 atomes d'halogènes, et/ou nitro, et r $R^8$ représente un groupe alkyle à chaîne droite ou ramifiée en $C_1$-$C_{12}$ ou cycloalkyle en $C_3$-$C_8$ lorsque tous les symboles X représentent le chlore.

5. Thioéthers bêta-céto-trihalogénométhyliques de formule

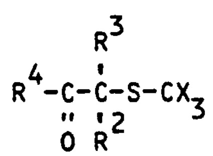

(IIc)

dans laquelle

$R^2$ et $R^3$ représentent chacun, indépendamment l'un de l'autre, l'hydrogène ou un groupe alkyle à chaîne droite ou ramifiée en $C_1$-$C_4$,

$R_4$ représente un groupe phényle qui peut porter un ou plusieurs substituants identiques ou différents choisis parmi les halogènes, les groupes alkyles en $C_1$-$C_4$, halogénoalkyle contenant 1 à 4 atomes de carbone et 1 à 5 atomes d'halogènes, alcoxy en $C_1$-$C_4$, halogénoalcoxy contenant 1 à 4 atomes de carbone et 1 à 5 atomes d'halogènes, alkylthio en $C_1$-$C_4$, halogénoalkylthio contenant 1 à 4 atomes de carbone et 1 à 5 atomes d'halogènes, et/ou nitro, et

X représente le fluor ou le chlore, l'un au moins des symboles X représentant le chlore.

6. Procédé de préparation des thioéthers bêta-céto-trihalogénométhyliques de formule

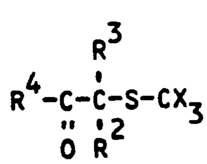

(IIc)

dans laquelle

$R^2$ et $R^3$ representent chacun, indépendamment l'un de l'autre, l'hydrogène ou un groupe alkyle à chaîne droite ou ramifiée en $C_1$-$C_4$,

$R^4$ représente un groupe phényle qui peut porter un ou plusieurs substituants identiques ou différents choisis parmi les halogènes, les groupes alkyle en $C_1$-$C_4$, halogénoalkyle contenant 1 à 4 atomes de carbone et 1 à 5

atomes d'halogènes, alcoxy en $C_1$-$C_4$, halogénoalcoxy contenant 1 à 4 atomes de carbone et 1 à 5 atomes d'halogènes, alkylthio en $C_1$-$C_4$, halogénoalkylthio contenant 1 à 4 atomes de carbone et 1 à 5 atomes d'halogènes, et/ou nitro,

X représente le fluor ou le chlore, l'un au moins des symboles X représentant le fluor,

caractérisé en ce que l'on fait réagir des cétones de formule

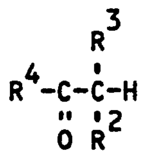

(III)

dans laquelle

$R^2$, $R^3$ et $R^4$ ont les significations indiquées ci-dessus avec des chlorures de trihalogénométhyl-sulfényle de formule

$Cl$-$SCX_3$ (IV)

dans laquelle

X a les significations indiquées ci-dessus, éventuellement en présence d'un diluant.

7. Thioéthers bêta-céto-trihalogénomethyliques de formule

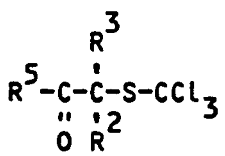

(IId)

dans laquelle

$R^2$ et $R^3$ représentent chacun, indépendamment l'un de l'autre, l'hydrogène ou un groupe alkyle à chaîne droite ou ramifiée en $C_1$-$C_4$ et

$R^5$ représente un groupe alkyle à chaîne droite ou ramifiée en $C_1$-$C_{12}$ ou cycloalkyle en $C_3$-$C_8$.

8. Procédé de préparation des thioéthers bêta-céto-trihalogénométhyliques de formule

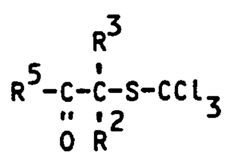

(IId)

dans laquelle

$R^2$ et $R^3$ représentent. chacun, indépendamment l'un de l'autre, l'hydrogène ou un groupe alkyle à chaîne droite ou ramifiée en $C_1$-$C_4$ et

$R^5$ représente un groupe alkyle à chaîne droite ou ramifiée en $C_1$-$C_{12}$ ou cycloalkyle en $C_3$-$C_8$, caractérisé en ce que l'on fait réagir des cétones de formule

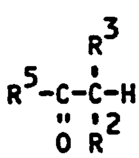

(V)

dans laquelle

$R^2$, $R^3$ et $R^5$ ont les significations indiquées ci-dessus, avec le chlorure de trichlorométhyl-sulfenyle de formule

$Cl$-$S$-$CCl_3$ (IVa)

éventuellement en présence d'un diluant inerte.